(19) Europäisches Patentamt
European Patent Office
Office européen des brevets

(11) Publication number: **0 041 283**
**B1**

(12) # EUROPEAN PATENT SPECIFICATION

(45) Date of publication of patent specification: **24.07.85**

(51) Int. Cl.⁴: **C 07 D 307/93**, C 07 C 61/40, A 01 N 53/00

(21) Application number: **81200491.9**

(22) Date of filing: **08.05.81**

(54) **Process for the preparation of dihalomethylene-lactones.**

(30) Priority: **30.05.80 GB 8017700**

(43) Date of publication of application:
**09.12.81 Bulletin 81/49**

(45) Publication of the grant of the patent:
**24.07.85 Bulletin 85/30**

(84) Designated Contracting States:
**CH DE FR GB IT LI NL**

(56) References cited:
**EP-A-0 003 666**
**DE-A-2 639 777**
**FR-A-2 396 006**

**AGRICULTURAL AND BIOLOGICAL CHEMISTRY, vo. 39, no. 7, May 1975, The Agricultural Chemical Society of Japan (JP) T. SUGIYAMA et al.: "Configurational Relationship between Substituents on Cyclopropane Ring of Pyrethroids and Their Insecticidal Toxicity" pages 1483-1488**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Kramer, Petrus Anthonius**
**Badhuisweg 3**
**NL-1031 CM Amsterdam (NL)**

(74) Representative: **Hunter, Keith Roger Ian et al**
**4 York Road**
**London SE1 7NA (GB)**

(56) References cited:
**CHEMISTRY LETTERS, 1979 Chemical Society of Japan (JP) K. KONDO et al.: "Stereospecific synthesis of cis-dihalovinyl-cyclopropanecarboxylic acid" pages 1185-1188**
**Fieser and Fieser: Reagents for Organic Synthesis 4, 390**
**Buehler, Pearson: Survey of Organic Syntheses 1, 84-85**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 041 283 B1

**Description**

The invention relates to a process for the preparation of halo-substituted lactones. These compounds can be employed as intermediates in the preparation of insecticides known as pyrethroids. These pyrethroids show remarkable activity against various insect pests and also possess a very low mammalian toxicity; such a combination of properties makes them useful as pesticides for crop protection. One of the most successful groups of pyrethroids includes esters of 2-(2,2-dihalovinyl)-3,3-dimethylcyclopropane carboxylic acid and it is known that esters based on the *cis*-form of this acid have even better insecticidal activity. This *cis*-dihalovinyl-acid may be represented as follows:-

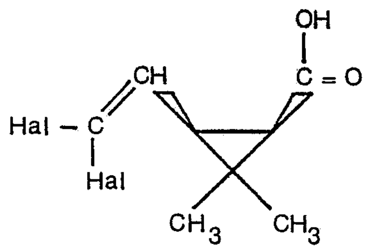

(A)

where Hal represents a halogen atom. Such a high level of activity has provided a stimulus to the search for new and economic routes to the *cis*-dihalovinyl-acid and it is an object of the present invention to provide a better process which can be employed in its synthesis.

The preparation of halo-substituted bicyclic lactone intermediates has been proposed already for the preparation of the dihalovinyl acid A. For instance, Chemistry Letters (Chemical Society of Japan) 1979, pages 1185—1188, describes the reaction

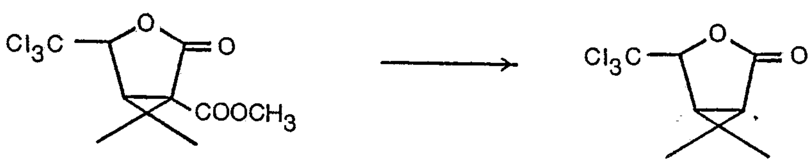

using LiCl and HMPA and having a yield of 37%. The trihalomethyl substituted bicyclic lactone product may be converted in one step to the methyl ester of the dihalovinyl acid A. Both this article and EP—A—3666 describe an alternative preparation of the trihalomethyl-lactone, involving the catalytic cyclization of 3-methyl-1-trihalomethyl-2-butenyl diazoacetate, which reaction has a yield of 35% if bromine is used as the halogen substituent and of 68% in the case of chlorine. Obviously both preparations may be improved yield-wise, and also, the use of azo-compounds is preferably avoided.

It has now been found that dihalomethylene-substituted bicyclic lactones may be used advantageously as intermediates in the preparation of A too, and that these intermediates may be obtained in surprisingly high yield from the corresponding trihalomethyl-substituted hydroxylactones by using phosphorus trihalide, zinc and a polar aprotic inert solvent.

Accordingly, the present invention concerns a process for the preparation of a dihalomethylene-lactone of general formula I

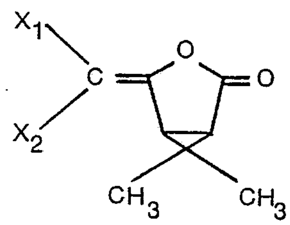

(I)

wherein $X_1$ and $X_2$ each individually represent a fluorine, chlorine or bromine atom, by reacting a hydroxylactone of general formula

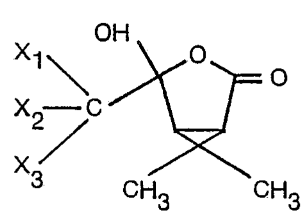

(III)

wherein $X_1$, $X_2$ and $X_3$ are individually chlorine, bromine or fluorine atoms, with a phosphorus trihalide and zinc in an inert polar aprotic solvent.

It should be noted that the introduction of a double bond by the elimination of a halogen atom and a hydroxy group from a halohyrodrin is known per se; for instance Fieser and Fieser, *Reagents for Organic Synthesis,* Volume 4, page 390, and Buehler and Pearson, *Survey of Organic Syntheses,* Volume 1, pages 84—85, describe the use of phosphorus oxychloride (phosphoryl chloride, $POCl_3$) and pyridine in the preparation of certain olefins, starting from iodohydrins. However, nor phosphorus trihalides nor zinc are advocated herein as reactants, and it could not be predicted that these reactants in combination with an inert aprotic polar solvent would effect the present elimination reaction in yields as high as 90%.

The dihalomethylene-lactones prepared according to the invention can be readily converted into the *cis*-dihalovinyl acids of formula A by, firstly, reacting the lactone with methanol in the presence of acid to produce a *cis*-dihaloacetyl-dimethyl-cyclopropane carboxylate methyl ester, which ester is broadly disclosed in DE—A—2.639.777 (as compound III therein), and secondly, converting this ester into the desired pyrethroid intermediate of formula A by employing procedures analogous to those described DE—A—2.639.777 (as described in scheme A therein). It has been established that pyrethroid insecticides based on this *cis*-dihalovinyl acid are much more insecticidally-active than the *trans*-dihalovinyl acids and thus the compounds prepared according to the invention, all of which can give rise to *cis*-dihalovinyl acids, are of considerable economic and agronomic importance.

The compounds prepared according to the invention may also be obtained as a by-product in the process claimed in our application GB—A—2076818, in which *cis*-caronic anhydride (the anhydride of *cis*-3,3-dimethylcyclopropane-1,2-dicarboxylic acid) is reacted under substantially anhydrous conditions with an alkali metal trihaloacetate.

The starting material of the process according to the present invention, can also exist in a *cis*-keto acid tautomeric form, and is claimed as such in said application GB—A—2076818; it can be prepared by methods analogous to those described in Journal of Organic Chemistry Vol. 32 (1967) pp. 2166—2171.

The substituents $X_1$, $X_2$ and $X_3$ in the hydroxy-lactone of formula III preferably represent chlorine atoms and the phosphorus trihalide is preferably phosphorus trichloride. Likewise, in the dihalomethylene-lactone of formula I, the substituents $X_1$, and $X_2$ preferably represent chlorine.

The inert polar aprotic solvent may be any solvent which possesses these properties, for example N-methylpyrrolidone, N,N-dimethylformamide or acetonitrile. The term "aprotic" as used herein denotes a solvent which is free from hydrogen atoms that are able to form hydrogen bonds with anions. This definition is in accordance with "Physical Chemistry of Organic Solvent Systems", edited by A.K. Corrington and T. Dickinson, Plenum Press (1973), pages 332 and 333. Preferably the solvent N,N-dimethylformamide is used.

The temperature range over which the process may be carried out is not critical, but temperatures in the range −20° to 100°C are often useful, temperatures in the range 0—100°C being preferred.

The molar ratio of the hydroxylactone III to the phosphorus trihalide can vary widely but a preferred range is 1:1 to 1:4. The preferred molar ratio of the hydroxylactone to zinc is in the range 1:1 to 1:4.

The present invention is now further illustrated by the following Example:-

Example

Preparation of 4-dichloromethylene-6,6-dimethyl-2-oxo-3-oxabicyclo[3.1.0]hexane and the corresponding dibromo compound

An NMR tube was charged with a 20/80 mixture of a hydroxylactone of formula:-

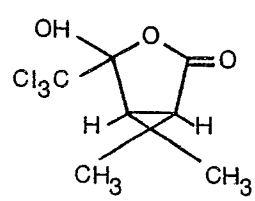

and the tautomeric *cis*-keto acid of formula:-

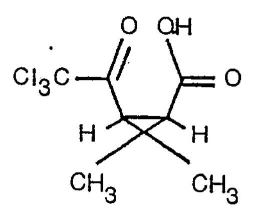

(78 mg, 0.30 mmol), 124 mg $PCl_3$ (0.90 mmol) and 0.4 ml $CDCl_3$. After 16 h at 50°C the solvent was evaporated and the oily residue was dissolved in 0.5 ml dimethyl formamide. Afterwards 39 ml Zn (0.60

mmol) was added at 20°C and the reaction mixture was shaken during five minutes and subsequently diluted with 1 ml water, extracted with 0.4 ml $CDCl_3$, the organic phase was washed three times with 1 ml water and analysed by GLC and NMR. Conversion: 100%; selectivity: 92%.
NMR: Varian EM—390 spectrometer.

Chemical shift δ in ppm from TMS solvent $CDCl_3$
A and B 1.21 (s, 3H) and 1.29 (s, 3H)
C 2.37 (d, 1H), $J_{CD}$ = 6 Hz
D 2.85 (d, 1H)

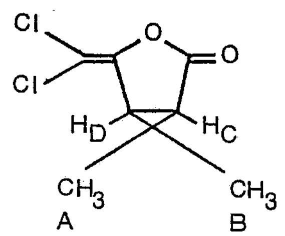

The following dibromo-methylene compound was also prepared

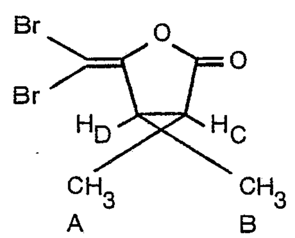

The NMR data was as follows:-
A and B 1.21 (s, 3H) and 1.28 (s, 3H)
C 2.42 (d, 1H), $J_{CD}$ = 6 Hz
D 2.86 (d, 1H)

**Claims**

1. Process for the preparation of a dihalomethylene-lactone of general formula I

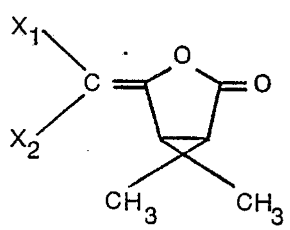

(I)

wherein $X_1$ and $X_2$ each individually represent a fluorine, chlorine or bromine atom, characterised in that a hydroxylactone of general formula

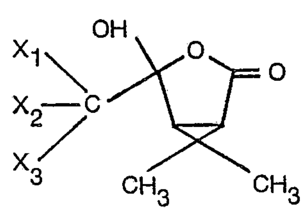

(III)

wherein $X_1$, $X_2$ and $X_3$ are individually chlorine, bromine or fluorine atoms, is reacted with a phosphorus trihalide and zinc in an inert polar aprotic solvent.

2. A process according to claim 1, wherein $X_1$, $X_2$ and $X_3$ in formula III represent chlorine atoms.

3. A process according to claim 1 or 2, wherein the phosphorus trihalide is $PCl_3$.

4. A process according to any one of claims 1 to 3, wherein the inert polar aprotic solvent is N,N-dimethylformamide.

**Patentansprüche**

1. Verfahren zur Herstellung eines Dihalogenmethylen-lactons der allgemeinen Formel

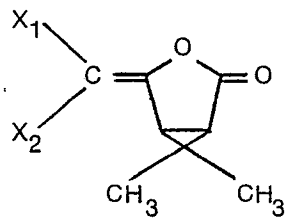

(I)

in der $X_1$ und $X_2$ jeweils ein Fluor-, Chlor- oder Bromatom bedeuten, dadurch gekennzeichnet, daß ein Hydroxylacton der allgemeinen Formel

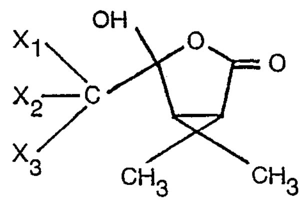

(III)

in der $X_1$, $X_2$ und $X_3$ jeweils Chlor-, Brom- oder Fluoratome bedeuten, umgesetzt wird mit einem Phosphortrihalogenid und Zink in einem inerten polaren aprotischen Lösungsmittel.

2. Verfahren nach Anspruch 1, wobei $X_1$, $X_2$ und $X_3$ in Formel III Chloratome bedeuten.

3. Verfahren nach Anspruch 1 oder 2, wobei das Phosphortrihalogenid $PCl_3$ ist.

4. Verfahren nach einem der Ansprüche 1 bis 3, wobei das inerte polare aprotische Lösungsmittel N,N-Dimethylformamid ist.

**Revendications**

1. Un procédé pour la préparation d'une dihalométhylène lactone de formule générale I

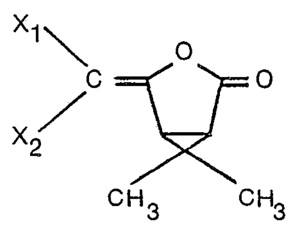

(I)

où $X_1$ et $X_2$ représentent chacun individuellement un atome de fluor, de chlore ou de brome, caractérisé en ce qu'une hydroxylactone de formule générale

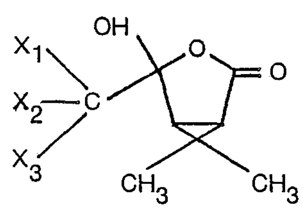

(III)

où $X_1$, $X_2$ et $X_3$ sont individuellement des atomes de chlore, de brome ou de fluor, est mise à réagir avec un trihalogénure de phosphore et du zinc dans un solvant aprotique polaire inerte.

2. Un procédé selon la revendication 1, dans lequel $X_1$, $X_2$ et $X_3$ dans la formule III représentent des atomes de chlore.

3. Un procédé selon la revendication 1 ou 2, dans lequel le trihalogénure de phosphore est $PCl_3$.

4. Un procédé selon l'une quelconque des revendications 1 à 3, dans lequel le solvant aprotique polaire inerte est du N,N-diméthylformamide.